# EUROPEAN PATENT APPLICATION

(11) **EP 4 104 874 A1**
(43) Date of publication of application: **21.12.2022**
(21) Application number: 22461575.7
(22) Date of filing: 20.06.2022
(51) Int. Cl.: A61L 27/32, A61L 27/18, A61L 27/50, A61L 27/54

(54) **HYDROXYAPATITE ANTIMICROBIAL AND OSTEOINDUCTIVE COATING AND METHOD OF PRODUCING SUCH A COATING**

(30) Priority: 20.06.2021 PL 43820921
(71) Applicant: Instytut Wysokich Cisnien Polskiej Akademii Nauk, 01-142 Warszawa (PL)
(72) Inventor: LOJKOWSKI, Witold, 02-792 Warszawa (PL); PIETRZYKOWSKA, Elzbieta, 07-130 Lochów (PL); ABOU AITAH, Khaled, 11843/0020 El-Salam, Cairo (EG); FUDALA, Damian, 03-017 Warszawa (PL); SZALAJ, Urszula, 03-042 Warszawa (PL); SWIDERSKA-SRODA, Anna, 00-738 Warszawa (PL); BIL, Monika, 01-493 Warszawa (PL); LOJKOWSKI, Maciej, 02-972 Warszawa (PL); SWIESZKOWSKI, Wojciech, 04-763 Warszawa (PL); NASILOWSKA, Justyna, 01-307 Warszawa (PL); SOKOLOWSKA, Barbara, 02-202 Warszawa (PL); WOZNIAK, Bartosz, 03-114 Warszawa (PL)
(74) Representative: Adamczyk, Piotr

(57) **Abstract**

The coating according to the invention is applied onto a surface of a medical implant's support substrate, consisting of nanocrystallites of synthetic hydroxyapatite with a molar ratio of calcium to phosphorus greater than 1.55 and less than 1.67, it is porous and the specific surface area of its material is at least 155 m²/g. The coat-forming hydroxyapatite is in the form of grains with an average size of at most 25 nm and at least 7 nm, is treated by ultrasonic cavitation and contains 4 to 6 wt% of structural water. The coating thickness ranges from 350 nm to 1000 nm.

## Description

The object of the invention is a thin coating of hydroxyapatite covering the surface of a medical implant saturated with an antimicrobial agent and a method of producing such a coating as well as a medical implant with such a coating

Medical implants, primarily orthopaedic and dental implants, are the subject of constant research and exploration intended to improve them. One problem which remains unresolved involves topical infections associated with bacterial contamination of the implant surface by bacteria such as *Staphylococcus, Enterobacteriacea,* and *Pseudomonas.* Bacteria and pathogens can form a biofilm on the surface of the implant, resulting in infection, inflammation, or even implant rejection. Gram-positive *Staphylococcus aureus* bacteria, for example, are capable of producing a biofilm on an implant within 18 to 24 hours of infection. Topical infections account for more than 10% of clinical complications that occur when a medical implant is inserted into the body. The initial several hours after implantation, with a maximum of the first 24 hours, are considered critical for the formation of biofilm on the surface of the implant. In many cases, it is advisable to administer an antibiotic preventively for a long time after implantation. There are implants on the market that release a long-lasting drug, such as sponges that release gentamicin over the course of several days. These solutions are achieved by introducing large amounts of antibiotic into the implants, for example, in the form of polymer sponges, ceramic layers, polymer coatings, and silver and zinc-oxide nanoparticle coatings. Many solutions to the long-term antimicrobial effects of the implant, lasting from a few days to several weeks, have been described in the literature. However, there is a strong need to equip implants with antimicrobial features during the first 24 hours to protect against topical infections. Calcium phosphate, in particular hydroxyapatite (HAp), has been used for many years as a coating material for implants, as it forms a highly biocompatible coating on them, stimulating the body to regenerate, mainly to rebuild bone tissue. However, there are cases where a coating of osteoinductive and osteointegrative hydroxyapatite also serves as a drug carrier.

The article by Townsend et al. titled, Antimicrobial peptide coatings for hydroxyapatite: electrostatic and covalent attachment of antimicrobial peptides to surfaces" [Journal of the Royal Society, Interface, 2017, 14(126), 20160657], describes a dual-coating implant with osteoinductive and antimicrobial properties, which is active against Gram-positive and Gram-negative bacteria for more than 12 months. The role of antimicrobial peptides attached to HAp is to inhibit bacterial growth and biofilm formation. The article by Yang et al. titled, "Vancomycin-chitosan composite deposited on post porous hydroxyapatite coated Ti6Al4V implant for drug controlled release" [Materials Science and Engineering: 2013, C, Vol. 33 (4): 2203-2212] discloses electrochemical deposition of a hydroxyapatite coating on a titanium implant, followed by electrolytic deposition of a vancomycin-chitosan composite, to provide antimicrobial effects to this implant. The hydroxyapatite coating has a porous structure 7.7 µm thick, and the antibiotic coating is an additional dense coating about 3 µm thick which contains from 150 to 550 µg/cm² of antibiotic. Vancomycin release profiles demonstrate a rapid release in the first hours (55%), followed by a steady release (20%) over 5 days, and a final release (25%) lasting more than 30 days. The microbicide-coated implant provides antimicrobial and bone anti-inflammatory effects, demonstrated in animal model studies.

Patent publication WO2018/042430A1 and related article by Geuli et al. titled, "Synthesis, coating, and drug-release of hydroxyapatite nanoparticles loaded with antibiotics" [Journal of Materials Chemistry B, 2017, 5(38), 7819-7830] discloses a single-step method for electrophoretic deposition of drug-loaded hydroxyapatite nanoparticles on titanium implants. The drug is added during the synthesis of hydroxyapatite, which is what controls the morphology of the powder. The average hydroxyapatite particle size, for example, with the drug ciprofloxacin (Cip), as measured by transmission electron microscopy (TEM), is 40 ± 12 nm. The aforementioned publications showed that the coating contained nanoparticles and gentamicin sulfate (Gs) or ciprofloxacin (Cip) in amounts of 12.5 wt% and 12.8 wt%, as determined by spectrophotometry. The results presented showed prolonged release of antibiotics, lasting up to 25 days for Gs (25%) and up to 10 days for Cip. In addition, 70% of Cip was released in the first 24 hours. *In vitro* antimicrobial tests of Cip- and Gs-HAp coatings showed effective inhibition of *Pseudomonas aeruginosa* bacteria growth. An HAp coating with a thickness of 9.3 to 24.9 µm and a surface roughness of 0.39 to 2.31 µm (depending on the antibiotic used) was used.

Patent publication CN107261202 describes the deposition of a hydroxyapatite coating using a deposition technique, lasting from 1 to 48 hours, on the surface of a modified titanium substrate with a nanotube structure. An HAp coating between 20 and 1,000 µm thick was obtained, which was then soaked in an antibiotic solution (vancomycin and/or gentamicin). The drug content ranged from 0.1 to 0.5 mg/cm² of the implant substrate surface. Antimicrobial efficacy of the coating against *Staphylococcus aureus* bacteria was demonstrated.

Patent publication CA2432284C discloses a calcium phosphate or hydroxyapatite coating containing an antibiotic therapeutic agent. The hydroxyapatite layer obtained by coating a metal implant three to five times by precipitating HAp from an aqueous solution. The coating thickness was about 1 µm for triple coating and 20 µm for quintuple coating. The antibiotic was released within 24 hours due to the nature of the interaction between antibiotics and hydroxyapatite, which involves physical adsorption. Note that micron-sized HAp coatings tend to crack.

Patent publication US9682170B2 discloses coating with hydroxyapatite nanoparticles (50 to 300 nm) and other nanoparticles of dental and orthopaedic implants with antimicrobial and osteointegrative properties. Coating is performed using the electrostatic spray method combined with a sintering process, a side effect of which is grain growth and loss of structural and surface water in HAp. The final thickness of the coatings produced ranged from 60 to 200 µm. The surface has an island-like microstructure and the roughness is about 250 µm, with nano- to micro-sized pores into which antimicrobial drugs or nanoparticles of other oxides (e.g., silver or zinc) can be introduced. The drug loading process can be achieved by immersion, embedding, or vapour deposition.

Patent publication US6730324B2 discloses coatings with HAp and microspheres as drug carriers. In the sol-gel process, a porous layer 10 to 1,000 µm thick is obtained by performing the coating multiple times. The drug used was amethopterin (5 wt%), which can be introduced by various means, such as into the pores of the layer or as microspheres (10-1,000 µm). The first release of the drug occurs after about 8 hours and lasts up to 60 hours. Furthermore, patent publication US006143948A discloses the coating of an implant with a thick layer with a porous structure and rough surface using a plasma deposition technique. The thickness of such a coating ranges from 1 to 50 µm.

Polyether ether ketone (PEEK) is also well-known in the medical field as an alternative material to metals for medical implants. It has high mechanical strength for a plastic, high chemical resistance, is biologically inert, can be easily machined, and has a modulus of elasticity similar to that of bone tissue. Like metal implants, PEEK implants are also coated with HAp to improve bone anchorage of the plastic implant.

Patent publication US20090276053A1 discloses the application of hydroxyapatite via a plasma spray process to the surface of a PEEK implant. The resulting hydroxyapatite coating is 45 to 100 µm thick and adheres strongly enough to the PEEK surface to ensure bone growth and/or bone tissue formation around the implant.

The article by Johansson et al. titled, "Nanosized Hydroxyapatite Coating on PEEK Implants Enhances Early Bone Formation: A Histological and Three-Dimensional Investigation in Rabbit Bone" [Materials, 2015, 8, 3815-3830] and the article by Meirelles L et al. titled, "Nano hydroxyapatite structures influence early bone formation" [J.Biomed. Mater. Res. Part A. 2008; 87:299-307] describe HAp coatings that enhance osteointegration of PEEK implants with a thickness of 20 to 40 nm. A suspension of 10-nm HAp particles was used to form the coating, in which the implant was immersed, and then the hydroxyapatite-coated implant was heat-treated at 550°C for 5 minutes under a nitrogen atmosphere.

Patent Publication No. RO132032B1 discloses ultrasonic fabrication of an antimicrobial coating of silver and zinc nanoparticles on an implant, although not a PEEK implant

Publication No. WO2016/178174A1 discloses cavitation coating of bone implants by immersing the plastic implant substrate in an aqueous suspension of hydroxyapatite particles, however, the publication does not provide any guidance on the possibility of soaking such implants in therapeutic substances.

The purpose of the invention is to obtain a thin i.e., less than 1-µm-thick hydroxyapatite coating on the external surface of an implant with the ability to adsorb antimicrobial substances in the amount of at least 0.8 mg/cm² of the implant surface, and then release at least 85% of the adsorbed substance at the implantation site within at most 24 hours after implantation.

This purpose is met by a coating according to the invention consisting of nanocrystallites of synthetic hydroxyapatite with a molar ratio of calcium to phosphorus greater than 1.55 and less than 1.67. The coating is characterized in that it is porous and the specific surface area of its material is at least 155 m²/g. The synthetic coat-forming hydroxyapatite is in the form of grains with an average size, measured by transmission electron microscopy or by X-ray powder diffraction (XRD), of at most 25 nm and at least 7 nm. Such hydroxyapatite is treated by ultrasonic cavitation and contains 4 to 6 wt% of structural water. The coating thickness ranges from 350 nm to 1000 nm.

In one of variants of the coating according to the invention, its pores are filled with an antimicrobial agent in the amount of at least 0.8 mg per one cm² of the surface of the implant support substrate.

In another variant of the coating according to the invention, the antimicrobial agent filling its pores is a β-lactam antibiotic active against Gram-positive and Gram-negative bacteria.

In another variant of the coating according to the invention, the antimicrobial agent is a second-generation cephalosporin.

In another option of the coating according to the invention, the material of its support structure is polyether ether ketone.

The coating according to the invention can be made according to any of the variants of the method according to the invention.

The method according to the invention involves applying synthetic hydroxyapatite to a support structure by immersing and immobilizing this structure in a suspension. This suspension is formed by a liquid dispersing phase, preferably water, and a dispersed phase in the form of grains with an average size of at most 30 nm of synthetic hydroxyapatite with a molar ratio of calcium to phosphorus of at least 1.55 and at most 1.67. The next step in this method is to induce the phenomenon of cavitation in the part of the suspension in contact with the support structure. The mass fraction of the dispersed phase in the suspension ranges from 0.01 to 2 wt%, and the cavitation state is induced using a sonotrode immersed in the suspension near the support structure. This sonotrode has a front surface that is vibrated at frequencies ranging from 18 kHz to 40 kHz. During the generation of a cavitation state, the distance between the sonotrode's front surface and the surface of the support structure is at most 200% of the diameter of its front surface. The method according to the invention is characterized in that a sonotrode with adjustable vibration amplitude is used, and the process of applying the hydroxyapatite grains involves at least three consecutive cycles. Each of these cycles consists of the preparation of a fresh hydroxyapatite suspension, cavitation interaction of this suspension with the support structure immersed in it, and drying. In each successive cycle, the amplitude of vibration of the front surface of the sonotrode is less than the amplitude of its vibration in the immediately preceding cycle, and the duration of the cavitation state in each cycle does not exceed 6 minutes, with the duration of cavitation in each successive cycle being no longer than the period of this cavitation in the cycle immediately preceding it.

In one of variants of the method according to the invention, the temperature of the suspension does not exceed 40°C.

In another variant of the method according to the invention, five cycles of hydroxyapatite grain application are used. The first cycle uses a sonotrode front surface vibration amplitude of 75% of the reference amplitude. In the second cycle, the vibration amplitude is 65% of the said reference amplitude, and in the third, fourth, and fifth cycles, a vibration amplitude of 55% of the reference amplitude is used. The duration of the cavitation state in the first cycle is six minutes, while in the next four cycles it is five minutes.

In another variant of the method according to the invention, the coating applied to the implant support structure is saturated with an aqueous solution of an antibiotic and then dried.

In another variant of the methods according to the invention, a salt of cefuroxime is used as an antibiotic.

In yet another variants of the method according to the invention, the cefuroxime salt is used in the form of a solution with a concentration of 45 mg/ml with a pH of 5.

An implant with a coating according to the invention, despite an HAp coating thickness of less than 1 µm, can adsorb over 1 mg of antimicrobial substance per 1 cm², which is more than twice as much as can be done by known implants with a much thicker hydroxypatite coating. The implant substrate material can be easily shaped, and thanks to the thin HAp coating, it does not fall off when the implant is manipulated during implant placement, such as by trimming and/or bending. No toxic reagents, necessary in known methods that require special activators, stabilizers, etc., are needed to create the hydroxyapatite coating. The obtained coating according to the invention provides the possibility of rapid (within a few hours) delivery of a large amount of antibiotic to the implant area, while the antibiotic remaining in the coating after this period, in the amount of 4 to 15% of its original amount (from 40 to 140 µg per 1 cm² of the surface of the substrate) ensures, even in the long-term, preservation of its concentration that is higher than the minimum inhibitory concentration (MIC), a phenomenon not encountered in known solutions. The coating according to the invention can be used to load the implant with various water-soluble drugs, without any restrictions, such as temperature sensitivity. Despite such a high content of the medicinal substance in the coating, it does not adversely affect the osteoinductive and osteointegrative properties of the hydroxyapatite coating.

The invention will be presented in more detail in the following exemplary embodiments. In these embodiments, two hydroxyapatite nanopowders with the trade names GoHAP1 and GoHAP3 were used, having the following properties:
- GoHAP 1 powder: the particles of this nanopowder have a grain size of 8±1 nm, a specific surface area of 250 ± 25 m²/g, and a density of 2.87±0.02 g/cm³;
- GoHAP 3 powder: the particles of this nanopowder have a grain size of 15 ± 1 nm, a specific surface area of 140 ± 14 m²/g, and a density of 2.94 ± 0.02 g/cm³, and contain 4 to 6 wt% of structural water.

The average grain size in both of these naopowders was determined by transmission electron microscopy (TEM) or indirectly from density and specific surface area (SSA), where the average grain size was calculated from SSA and density, assuming that all HAP particles are spherical and identical. The density of the nanopowders was determined using a helium pycnometer (Micromeritics AccuPyc II 1340), at 24 ± 2°C, according to ISO 12154:2014, while their specific surface areas were determined by BET isotherm analysis using the Micromeritics Gemini 2360 device according to ISO 9277:2010. Prior to density and SSA measurements, the powders were dried at 150°C for 2 hours in a constant flow of helium in a Micromeritics FlowPrep 060 desorption station.

Square plates of polyether ether ketone (PEEK), measuring 1 × 1 cm and 2 mm thick, were used as the support structure for the implants. Prior to the hydroxyapatite coating process, the surface of each such plate was rinsed with ethanol in a water bath for 15 minutes using an ultrasonic scrubber. The source of the hydroxyapatite was an aqueous suspension of one of the nanopowders described above, with the proportion of the dispersed phase in the total prepared suspension being 0.1 wt%. The implant substrate was coated with hydrosyapatite particles according to the method disclosed in publication WO2016/178174A1, that is, by immersing and immobilizing the substrate in the prepared suspension and subjecting it to an ultrasonic wave with a frequency of 20 kHz and a power intensity of 12 W/cm², i.e., under conditions that ensure the occurrence of ultrasonic cavitation. To produce this cavitation, a titanium ultrasonic sonotrode with a front surface diameter of 13 mm was used, powered by a VCX750 generator (Sonics, Newtown, CT, USA) operating at 750 W. The optimal distance between the coated support structure and the vibrating sonotrode front surface is 100% of the diameter of the sonotrode front surface and should not exceed 200% of this diameter. Thus, in the embodiments described below, the optimal distance will be 15 mm, while the acceptable distance will be no more than 26 mm. The generator powering the sonotrode allows the sonotrode's front surface vibration amplitude to be adjusted, which makes it possible to change the strength of the cavitation produced in order to control the process of coating the supporting structure with hydroxyapatite. The temperature of the suspension was maintained between 28 and 30°C using a cooling system mounted on the walls of the tank in which the coating was carried out.

### Example 1

### Implant with lamellar PEEK support structure coated with five layers of the GoHAP 3 nanopowder

As a preliminary step, an aqueous suspension of GoHAP 3 nanopowder particles was prepared with a calcium-to-phosphorus ratio of 1.61, as determined by ICP-OES optical emission spectrometry. In this example, hydroxyapatite was applied to the aforementioned support structure in five cycles, each of which consists of the preparation of a fresh hydroxyapatite suspension, cavitation interaction of this suspension with the support structure immersed in it, and drying. In the first cycle, cavitation coating lasted six minutes with the amplitude of the sonotrode's vibration front set at 75% of the maximum value. In the second cycle, the amplitude of the sonotrode's vibration front was reduced to 65% of the maximum value and ultrasound was generated for five minutes. In each of the three subsequent cycles, the cavitation interaction also lasted for five minutes each, but the amplitude of the sonotrode's vibration front was limited to 55% of the maximum value.

After the coating process, the finished implant was removed, washed with deionized water, and dried at room temperature.

A spotting method was used to saturate the applied hydroxyapatite coating with the drug. A cefuroxime salt solution with a concentration of 45 mg/ml and a pH of 5 was prepared. The drug was applied using a pipette with a droplet size equivalent to 50 µl. After spotting, the implant surface was dried at room temperature, then the implant was rinsed over 5 seconds in a bath of deionized water stirred with a magnetic stirrer at 300 rpm, and dried again at room temperature. The drug spotting procedure was repeated three times.

In this embodiment, a bioactive hydroxyapatite coating was obtained on the surface of the PEEK support structure, characterized by:
- thickness of 760 ± 50 nm;
- coverage of more than 90% of the surface of the support structure;
- porosity with an average pore size of 104 ± 34 nm;
- wetting angle of 54 ± 6°;
- the amount of adsorbed drug of 1 mg for each cm² of the surface of the implant support structure;
- the amount of drug released from this coating in the first 24 hours: at least 85% of the amount adsorbed.

The thickness of the hydroxyapatite coating and its porosity were determined using an Ultra Plus (Zeiss, Jena, Germany) scanning electron microscope (SEM). The samples were broken in liquid nitrogen and their cross-section surface was examined. The obtained SEM images were analysed using specialized Gwyddion software.

The pore size distribution of the coatings was analysed in Excel calculation software using SEM images.

Wetting angle was determined with an OCA 20 (DataPhysics Instruments GmbH, Filderstadt, Germany). Measurement was made for deionized water, and specialized software was used for the analysis.

The amount of adsorbed drug was determined by extracting it into the medium and by spectrophotometric analysis. For this purpose, an implant with an antimicrobial coating of 1 cm² was placed in a glass bottle containing 3 ml of deionized water. It was subjected to sonication for 15 min (Elma sonicator with water bath, Germany) to wash out the drug, thus obtaining a solution for testing, which was then analysed. A total of 6 ml of solution was taken from each implant, which was subjected to UV-Vis analysis after filtering using a syringe filter (0.45 µm). Quantitative analysis was performed at 275 nm wavelength on a UV-Vis Evolution^{™} 60 spectrophotometer (Thermo Scientific, Pittsburgh, PA, USA) using a standard curve prepared for Cef. Each measurement to determine the amount of Cef was repeated five times.

### Study of release kinetics

The antimicrobial agent-coated implant was placed in a glass bottle and 3 ml of PBS buffer was added, then placed in an immersion circulator with a multi-position stirrer at 37°C and 150 rpm (IKA Poland, Warsaw). At time points from 2 h to 150 h, PBS solutions were taken and the antimicrobial implant was placed in a new PBS solution. The collected PBS solutions from different time points were stored in vials at 4°C. The PBS solutions were subjected to UV-Vis Evolution^{™} 60 analysis at a wavelength of 275 nm. The concentration of Cef at each time point was determined using a previously prepared calibration curve. Three measurements were taken for each sample. On this basis, it was determined that 86% of the drug was released within the first 24 hours.

### Example 2

### Implant with lamellar PEEK support structure coated with five layers of the GoHAP 1 nanopowder

The GoHAP 1 nanopowder with a calcium/phosphorus ratio of 1.65 was used to coat the lamellar support structure of the implant. Five ultrasonic coating cycles analogous to the first example were performed, obtaining a hydroxyapatite coating 478 ± 20 nm thick, followed by spotting with an antimicrobial drug as in the previous embodiment. The bioactive coating adsorbed the drug at a rate of 1.095 ± 0.130 mg/cm² of the supporting structure's surface. The wetting angle of the resulting coating was 64 ± 4°.

### Bactericidal test

The implant obtained in this embodiment was subjected to *in vitro* bactericidal testing using the spherical inhibition method. The assessment was conducted against S. aureus ATCC 25923. In the experiment, 100 µL of overnight culture (~108 cfu/ml) was performed in Tryptone Soy Broth (TSB; Biokar Diagnostics, Beauvais, France) and then transferred to Tryptone Soy Agar (TSA; Biokar Diagnostics). An implant with an antimicrobial coating containing Cef was placed on prepared agar plates and incubated at 37°C. A plate with S. aureus ATCC 25923 alone and a plate with S. aureus ATCC 25923 and an implant with a coating of GoHAP without antibiotic were incubated as controls. Zones of inhibition were measured every 24 hours for 11 days. The tests were performed three times. It was shown that the diameter of the zone of inhibition after 24 h was 45.0 ± 2.0 mm. It was observed that the zones were the same after 5 days of incubation and 11 days of incubation. Which proves the effectiveness of the antimicrobial coating for up to 11 days.

Broth dilution tests were also conducted using the cultures in TSB medium described earlier. Bacterial viability was determined by measuring optical density (OD) at a wavelength of 600 nm using a Nanodrop ND-100UV/Vis instrument (NanoDrop Technologies, USA). For this purpose, 10 µl of overnight culture of S. aureus ATCC 25923 in TSB was transferred to 3 ml of TSB and incubated at 37°C for 10 hours. Bacterial optical density tests were performed at time points every 14 hours for 7 days. The experiments were conducted three times. TSB with S. aureus ATCC 25923 alone and S. aureus ATCC 25923 and a GoHAP-coated implant without Cef were used as control samples. After each time point, implants and control samples were placed in a new 3 ml TSB solution with 10 µl of S. aureus ATCC 25923. The broth test showed the efficacy of the cefuroxime antimicrobial agent-coated implant against Staphylococcus aureus for 168 hours.

### Example 3

### Implant with lamellar PEEK support structure coated with two layers of the GoHAP 1 nanopowder

The GoHAP 1 powder was used to create the hydroxyapatite suspension, the same as in second example. Two coating cycles were used, with the first cycle generating ultrasound for six minutes using a sonotrode whose vibration amplitude of its front surface was limited to 75% of its maximum value, and the second cycle generated ultrasound for five minutes, with the vibration amplitude of the sonotrode's front surface set at 65% of its maximum value. As a result, an implant coated with a hydroxyapatite layer 368 ± 17 nm thick and with a wetting angle of 69 ± 5° was obtained.

### Example 4

### Implant with lamellar PEEK support structure coated with three layers of the GoHAP 3 nanopowder

The nanopowder used was the same as in the first example. Hydroxyapatite was applied to the lamellar support structure described above in three cycles. In the first cycle, the ultrasonic impact on the implant support structure lasted six minutes using a sonotrode in which the amplitude of the front surface vibration was set at 75% of the maximum value. In the second cycle, the vibration amplitude of the sonotrode front surface was reduced to 65% of the maximum value and ultrasound was generated for five minutes, while in the third cycle, the vibration amplitude of the sonotrode front surface was reduced to 55% of the maximum value and ultrasound was also generated for five minutes. Consequently, a hydroxyapatite coating with a thickness of 368 ± 32 nm and a wetting angle of 55 ± 6° was obtained.

## Claims

1. A hydroxyapatite antimicrobial and osteoinductive coating applied onto a surface of a medical implant's support substrate, consisting of nanocrystallites of synthetic hydroxyapatite with a molar ratio of calcium to phosphorus greater than 1.55 and less than 1.67, **characterized in that** it is porous and the specific surface area of its material is at least 155 m²/g, the synthetic coat-forming hydroxyapatite is in the form of grains with an average size, measured by transmission electron microscopy or by X-ray powder diffraction (XRD), of at most 25 nm and at least 7 nm, is treated by ultrasonic cavitation and contains 4 to 6 wt% of structural water, and the coating thickness ranges from 350 nm to 1000 nm.

2. The coating according to claim 1, **characterized in that** its pores are filled with an antimicrobial agent in the amount of at least 0.8 mg per one cm² of the surface of the implant support substrate.

3. The coating according to claim 2, **characterized in that** the antimicrobial agent filling its pores is a β-lactam antibiotic active against Gram-positive and Gram-negative bacteria.

4. The coating according to claim 1 or 2 or 3, **characterized in that** the antimicrobial agent is a second-generation cephalosporin.

5. The coating according to claim 1 or 2 or 3 or 4, **characterized in that** the material of its support structure is polyether ether ketone.

6. The coating according to claim 1 or 2 or 3 or 4 or 5, **characterized in that** it is produced by the method according to any of claims 7 to 12.

7. A method of producing a hydroxyapatite antimicrobial and osteoinductive coating, which involves applying synthetic hydroxyapatite to a support structure by immersing and immobilizing this structure in a suspension, and this suspension is formed by a liquid dispersing phase, preferably water, and a dispersed phase in the form of grains with an average size of at most 30 nm of synthetic hydroxyapatite with a molar ratio of calcium to phosphorus of at least 1.55 and at most 1.67, and by inducing the phenomenon of cavitation in the part of the suspension in contact with the support structure, the mass fraction of the dispersed phase in the suspension ranges from 0.01 to 2 wt%, and the cavitation state is induced using a sonotrode immersed in the suspension near the support structure and having a front surface that is vibrated at frequencies ranging from 18 kHz to 40 kHz, and during the generation of a cavitation state, the distance between the sonotrode's front surface and the surface of the support structure is at most 200% of the diameter of its front surface, **characterized in that** a sonotrode with adjustable vibration amplitude is used, and the process of applying the hydroxyapatite grains involves at least three consecutive cycles, and each of these cycles consists of the preparation of a fresh hydroxyapatite suspension, cavitation interaction of this suspension with the support structure immersed in it, and drying, and in each successive cycle, the amplitude of vibration of the front surface of the sonotrode is less than the amplitude of its vibration in the immediately preceding cycle, and the duration of the cavitation state in each cycle does not exceed six minutes, with the duration of cavitation in each successive cycle being no longer than the period of this cavitation in the cycle immediately preceding it.

8. The method according to claim 7, **characterized in that** the temperature of the suspension does not exceed 40°C.

9. The method according to claim 7 or 8, **characterized in that** five cycles of applying hydroxyapatite grains are used, and the first cycle uses a sonotrode front surface vibration amplitude of 75% of the reference amplitude, and in the second cycle, the vibration amplitude is 65% of the said reference amplitude, and in the third, fourth, and fifth cycles, a vibration amplitude of 55% of the reference amplitude is used, and the duration of the cavitation state in the first cycle is six minutes, while in the next four cycles it is five minutes.

10. The method according to claim 7 or 8 or 9, **characterized in that** the coating applied to the implant support structure is saturated with an aqueous solution of an antibiotic and then dried.

11. The method according to claim 10, **characterized in that** a salt of cefuroxime is used as an antibiotic.

12. The method according to claim 11, **characterized in that** the cefuroxime salt is used in the form of a solution with a concentration of 45 mg/ml with a pH of 5.
